# EUROPÄISCHE PATENTSCHRIFT

(11) **EP 3 684 448 B1**
(45) Veröffentlichungstag und Bekanntmachung des Hinweises auf die Patenterteilung: **01.06.2022**
(21) Anmeldenummer: 18804506.6
(22) Anmeldetag: 19.10.2018
(51) Int. Cl.: A61M 11/00, A61M 15/00

(54) **AUTOMATISCHE MEDIKAMENTENDOSIERUNG BEI VERNEBLERN**
AUTOMATIC DOSING OF MEDICAMENTS IN ATOMIZERS
DOSAGE AUTOMATIQUE DE MÉDICAMENTS DANS DES NÉBULISEURS

(30) Priorität: 23.10.2017 DE 102017124742
(43) Veröffentlichungstag der Anmeldung: 29.07.2020
(73) Patentinhaber: NEBU-TEC med. Produkte Eike Kern GmbH, 63820 Elsenfeld (DE)
(72) Erfinder: KERN, Stefan, 63820 Elsenfeld (DE)
(74) Vertreter: Pöhner, Wilfried Anton
(86) Internationale Anmeldenummer: PCT/DE2018/100859
(87) Internationale Veröffentlichungsnummer: WO 2019/080964

(56) Entgegenhaltungen:
- EP-A1- 1 205 198
- DE-A1-102015 101 091
- DE-A1-102016 108 250
- DE-U1-202011 110 785
- DE-U1-202015 102 538
- US-A1- 2003 146 300
- US-A1- 2012 216 800
- US-A1- 2016 193 434
- US-A1- 2017 232 211

## Beschreibung

Vorliegende Erfindung befasst sich mit einer Verneblereinheit mit einem Medikamentenreservoir zur Verwendung in einem Verneblergerät, umfassend einen Aerosolerzeuger zur Zerstäubung einer Medikamentenflüssigkeit mit einer Zuführungsseite, die sich in Kontakt mit der dort vorhandenen Medikamentenflüssigkeit befindet und einer Ausgangsseite, auf der der gebildete Nebel abgegeben wird, und der zwischen einem zuführungsseitigen Dichtring und einer ausgangseitigen Haltestruktur gehalten ist, ein Gehäuse, welches Aerosolerzeuger, Dichtring und Haltestruktur einschließt und ein in das Gehäuse auf der Zuführungsseite der Membran integriertes Medikamentenreservoir, welches durch eine dicht schließende Kappe verschließbar ist.

Um Atemwegs- und Lungenerkrankungen zu behandeln muss ein wirksames Medikament möglichst vollständig bis an den Ort der Erkrankung gebracht werden, das heißt bis in die Bronchien, Alveolargänge oder sogar in die Lungenbläschen. Hierzu wird üblicher Weise ein Medikament als Lösung vorbereitet, und mittels eines Verneblers fein zerstäubt bevor es durch den Patienten inhaliert wird.

Heute eingesetzte Vernebler, welche auch als mobile Geräte erhältlich sind, bestehen aus vier wesentlichen Komponenten: Das Herz des Verneblers ist die sogenannte Verneblereinheit, welche eine mikroperforierte Membran enthält, die mittels eines Schwingungserzeugers, üblicherweise ein Piezokristall, in schnelle Schwingungen versetzt wird, üblicherweise im Ultraschallbereich, wodurch die direkt an die Membran anliegende Medikamentenlösung in die mikrometergroßen Löcher der Membran hineingedrückt und auf der Ausgangsseite der Membran als ein Nebel von feinen Tröpfchen abgegeben wird. Üblicherweise ist die Membran umfänglich mit dem kreisringförmigen Schwingungserzeuger direkt, oder indirekt über eine Trägerplatte, mechanisch zu einer zusammenhängenden Einheit, dem Aerosolerzeuger, gekoppelt. Auf die Verneblereinheit wird entweder ein Schlauch mit Mundstück oder direkt ein Mundstück aufgesetzt. Des Weiteren gehört zu einem Vernebler eine Steuereinheit, welche die zur Ansteuerung des Schwingungserzeugers nötigen Signale erzeugt. Auf der Zuführungsseite der Membran, in die Verneblereinheit integriert befindet sich üblicherweise ein Reservoir für die Medikamentenlösung, welches einen ausreichend dimensionierten Hohlraum bereitstellt, welcher mittels einer Kappe fest verschließbar ist.

Das Reservoir muss ausreichend dimensioniert sein, um verschiedene Medikamentenmengen aufnehmen zu können. Bei jeder Inhalation ist es je nach Art und Schwere der Erkrankung und je nach Medikament eine unterschiedliche Medikamentendosis nötig, welche üblicherweise sehr genau eingehalten werden sollte. Darum stellt sich das Problem, wie diese exakte Dosierung sichergestellt werden kann.

Dies könnte zum einen dadurch geschehen, dass aus einem größeren Container eine kleinere, für die Inhalation benötigte Dosis abgemessen und diese dann in das Medikamentenreservoir gefüllt wird. Dies führt jedoch zu der Schwierigkeit, dass bei Einsatz durch nicht entsprechend geschulte/geübte Personen diese Dosierung von Kleinstmengen sehr fehlerbehaftet und ungenau ist oder die benötigten Hilfsmittel erst gar nicht zur Verfügung stehen.

Eine weitere Möglichkeit sieht vor, die Dosierung elektronisch zu regeln, indem die Steuereinheit die vom Aerosolerzeuger abgegebene Flüssigkeitsmenge überwacht. Dies ist im Prinzip dadurch möglich, dass die elektrischen und/oder mechanischen Eigenschaften des Schwingungserzeugers, wie beispielsweise verbrauchte Leistung oder die Resonanzfrequenz, von der Höhe des anliegenden Flüssigkeitsspiegels anhängig ist. Somit kann bei bekannter Form des Medikamentenreservoirs durch Überwachung von Strom und Spannung auf den anliegenden Druck geschlossen werden und aus dem Vergleich des aktuellen Drucks mit dem bei Inhalationsbeginn anliegenden Druck auf die schon ausgegebene Flüssigkeitsmenge ermittelt werden. Diese Methode ist jedoch auch mit größeren Unsicherheiten behaftet, sodass sie sich nicht für eine wirklich exakte Dosierung eignet.

Die Offenlegungschrift DE 10 2016 108 250 A1 stellt eine Verneblereinheit mit Medikamentenreservoir zur Verwendung in einem Vernebler vor, bei der eine Kappe des Medikamentenreservoirs einen Verdränger aufweist, der in das Innere des Reservoirs reicht so dass ein Restvolumen einer zu verabreichenden Medikamentendosis entspricht.

Die veröffentlichte Patentanmeldungsschrift US 2016/0193434 A1 zeigt ein Dosiersystem für eine Inhalationsvorrichtung, bei der ein zylinderförmiger Verdränger einer Kappe eines Medikamentenreservoirs bei eintauchen in eine Füllkammer eine fest definierte Medikamentendosis in eine darunterliegende Verneblungskammer gepresst wird, von wo aus das Medikament aerosolisiert wird. Für den Verdränger wird ein vergleichsweise flexibles Material, wie etwa Silikon, oder, sofern die Füllkammer aus einem flexiblen Material, etwa Silikon besteht, ein vergleichsweise hartes Material vorgeschlagen.

Die Gebrauchsmusterschrift DE 20 2015 102 538 U1 schlägt vor, einen Schwingungserzeuger und ein Stützaggregat eines Zerstäubers mittels einer Silikonhaftschicht an einem Gehäuse des Zerstäubers zu befestigen und mit einem Silikonpolster abzudecken.

Die Offenlegungsschrift DE 10 2015 101 091 A1 offenbart ein piezoelektrisches Verneblungsmodul mit einer Doppelluftkammer und einer gegenüber einem bloßen Dichtelement aus Gummi oder einem anderen Elastomer verbesserten Abdichtung.

In der Gebrauchsmusterschrift DE 20 2011 110 785 U1 ist ein Aerosolgenerator vorgestellt, bei dem ein Schwingungsgenerator beidseitig durch Dichtringe gehalten wird.

Das Dokument US 2017/0232211 A1 betrifft ein Flüssigkeitsreservoir für einen Aerosolerzeuger mit einer Flüssigkeitskammer und einer Öffnung um eine Flüssigkeit aus der Kammer nach außen zu leiten und einer Kappe zum Verschließen der Kammer. In manchen Ausführungsformen weißt die Kappe ein zylinderförmiges Verbindungselement mit einem Kragen an einem unteren Ende auf, wobei der Kragen ein Teilvolumen der Kammer, welches mit der Öffnung kommuniziert von einem Teilvolumen abtrennt, welches nicht mit der Öffnung in Verbindung steht.

Die veröffentlichte Europäische Anmeldung EP 1 205 198 A1 beschreibt eine Aerosolmedikamentenausgabevorrichtung welche eine abgemessene Dosis eines Medikaments abgibt, indem das Volumen einer ersten Kammer eines Reservoirs kontrolliert wird, wobei die erste Kammer eine flexible Teilfläche aufweist. Die veröffentlichte Anmeldung US 2003/0146300 A1 zeigt einen Vernebler mit einem Vernebelungsmittel und einer Abmesskammer für ein Flüssigmedikament und einer zweiten Kammer zum Auffangen von überschüssigem Medikament.

In der Anmeldeschrift US 2012/0216800 A1 ist ein Medikamentenabgabeapparat mit einem Reservoir für ein Flüssigmedikament und eine damit gekoppelte Abmessvorrichtung mit einer oberen und einer unteren Kammer offenbart.

Vor diesem Hintergrund stellt sich vorliegende Erfindung die Aufgabe, eine Verneblereinheit mit Medikamentenreservoir zu entwickeln, welche eine exakte Abmessung und Bereitstellung einer bei einer Inhalation zu verwendende Medikamentendosis auch für ungeübte beziehungsweise nicht über die entsprechenden Hilfsmittel verfügende Personen ermöglicht und die Verschwendung potentiell wertvoller Medikamentenflüssigkeit vermeidet.

Gelöst wird diese Aufgabe durch eine Verneblereinheit mit Medikamentenreservoir nach Anspruch 1 oder 2. Das Wesentliche hierbei ist, dass in die Verschlusskappe des Reservoirs ein Verschlusszapfen integriert wird, der das Gesamtvolumen des Reservoirs in zwei getrennte, also nicht in fluider Kommunikation stehende, Teilvolumina unterteilt, von denen eines auf einer Seite vom Aerosolerzeuger begrenzt wird und ein Volumen aufweist, welches einer zu inhalierenden Medikamentendosis entspricht.

Trotz der Bezeichnung 'Verschlusszapfen' ist die Form des in die Kappe integrierten oder an ihr angebrachten oder befestigten Körpers im Rahmen vorliegender Erfindung grundsätzlich nicht notwendig zapfen- oder zylinderartig. Im Sinne einer Minimierung des durch den Zapfen verdrängten Volumens ist jedoch eine möglichst schlanke Mittelsektion zwischen einer oberen Befestigung an oder in der Kappe, und dem unteren, den Verschluss bewirkenden Flächenteil des Verschlusszapfens sinnvoll.

Um dabei zu vermeiden, dass der Zapfens beim Verschließen der Kappe, bei dem ja die kooperierenden Teilflächen von Zapfen und Reservoir aufeinander gepresst werden, womit immer eine gewisse axiale Stauchung des Zapfens einhergeht, er sich in ungewollter Weise verformt, etwa eine Biegung entsteht, wird vorgeschlagen, Merkmale vorzusehen, die diesem entgegenwirken. Dies kann in Form von Versteifungsmerkmalen, wie Längsrippen oder -schienen realisiert werden. Alternativ oder zusätzlich können auch der Erhaltung der Form des Zapfens dienende Merkmale wie quer zur Längsrichtung verlaufende Ringe oder Wülste vorgesehen sein.

Die Verwendung der erfindungsgemäßen Verneblereinheit erfolgt hierbei so, dass zunächst aus einem Transportbehälter ein Medikament in das Reservoir eingeführt wird, bis mindestens die für eine einmalige Inhalation benötigte Dosis darin vorhanden ist. Sodann wird die Verschlusskappe des Medikamentenreservoirs aufgesetzt und befestigt, wobei durch den Verschlusszapfen automatisch vom Rest des Reservoirs ein an den Aerosolerzeuger grenzendes Teilvolumen abgetrennt wird, wodurch sichergestellt ist, dass die dem Aerosolerzeuger unmittelbar zuführbare, d.h. aerosolisierbare Medikamentenmenge genau der benötigten Dosis entspricht. Dann wird an die Verneblereinheit gegebenenfalls die Steuerung und das Mundstück angeschlossen und die Inhalation begonnen. Diese kann fortgesetzt werden bis die Steuereinheit eine vollständige Leerung des Reservoirs feststellt.

Der Vorteil der Verneblereinheit mit automatischer Dosierung nach vorliegender Erfindung besteht darin, dass auf sehr einfache Weise ein schnelles und exaktes Abmessen einer benötigten Medikamentendosis ermöglicht ist. Weder benötigt ein Anwender Hilfsmittel zur Dosierung wie sterilisierte Pipetten und Überträgergefäße, nach muss auf eine ungenaue elektronische Messung zurückgegriffen werden.

Um verschiedene Medikamentdosen abmessen zu können, werden in der einfachsten Ausführung vorliegender Erfindung einfach Verschlusskappen mit verschieden großen Verschlusszapfenn bereitgestellt. Da es sich bei den Kappen üblicherweise um relativ einfach herzustellende Plastikteile handelt, und die üblicherweise bei Inhalationen verabreichten Medikamentendosen eine diskrete Menge von Werten umfassen, ist dies ohne allzu großen Aufwand möglich.

Eine elegantere Lösung besteht jedoch darin, den Verschlusszapfen in seinem Volumen variabel zu gestalten. Dies ist zum Beispiel dadurch möglich, dass die Kappe zweiteilig ausgeführt wird: ein kreisringförmiger Teil mit einem Loch in der Mitte und einem umgebogenen äußeren Rand mit dem die Verbindung zum Medikamentenreservoir hergestellt wird, sowie ein in die Mitte des ersten Teil einschraubbares zylinderförmiges zweites Teil, welches als Verschlusszapfen dient. Durch die Schraubverbindung kann ein Verwender einstellen, wie weit der Verschlusszapfen in das Reservoirinnere ragt, und somit wieviel Restvolumen dort verbleibt. Dies wird dem Verwender dadurch erleichtert, dass auf der Außenseite des einschraubbaren Verschlusszapfens eine Volumenskala aufgezeichnet ist, die das noch vorhandene Restvolumen anzeigt. Um einen vollständig (flüssigkeits-)dichten Verschluss zu erreichen, kann das Gewinde mit entsprechenden Gummidichtungen versehen werden, oder es wird auf der Unterseite des ersten Teils eine elastische, flüssigkeitsdichte Membran vorgesehen, die beim Einschrauben des zweiten Teils gedehnt wird, wobei dann das oberhalb dieser Membran vorhandene, vom Inneren des Reservoirs abgetrennte Volumen das eigentliche Verschlusszapfenvolumen bildet.

-Da es sich bei manchen durch inhalativ applizierten Medikamenten um sehr teure Substanzen handelt, ist es vorteilhaft und wirtschaftlich angebracht, nicht benötigte Medikamentenlösung aufzufangen, sodass sie zu einer späteren Verwendung zur Verfügung steht. Dies ist dank der hier vorgestellten Ausgestaltung von Medikamentenreservoir und Kappe mit Verschlusszapfen möglich.

Die Verwendung der erfindungsgemäßen Verneblereinheit mit Medikamentenreservoir ist also für die Applikation einer ersten Inhalationsdosis wie oben beschrieben. Die erfindungsgemäße Verneblereinheit bietet jedoch zusätzlich die Möglichkeit, überzählige Medikamentenflüssigkeit wieder zu verwenden. Denn beim Einfüllen des Medikaments in das Reservoir und Aufsetzen der Kappen wird automatisch eine Abtrennung zwischen einem ersten, von der Zerstäubermembran, bzw. dem Aerosolerzeuger begrenzten Teilvolumen und einem zweiten, den Rest des Medikamentenreservoirs umfassenden, Volumen sichergestellt, wobei nicht benötigtes Medikament in letzteres hinein verdrängt wird. Nach Beenden des ersten Inhalationsvorgangs kann auf sehr einfach Art und Weise eine zweite Dosis abgemessen werden, indem nämlich einfach die Kappe ganz oder teilweise abgeschraubt oder abgenommen oder verdreht wird, sodass der Form- beziehungsweise Flächenschluss einer Seite des Verschlusszapfens mit der Innenwand des Reservoirs aufgehoben wird und Medikamentenflüssigkeit von dem zweiten in das erste Teilvolumen nachströmen kann. Ist dies vollständig erfolgt, so kann der Deckel wieder fest aufgesetzt und verschraubt und ein zweiter Inhalationsvorgang durchgeführt werden.

Der Vorteil der erfindungsgemäßen Verneblereinheit ist also, dass möglicherweise sehr teure Medikamentenflüssigkeit nicht verschwendet wird, sondern für die weitere Therapie zu Verfügung steht.

Erfindungswesentlich ist also, den Verschlusszapfen, und den Innenraum des Reservoirs so auszugestalten, dass bei vollständig aufgesetzter und verschlossener Kappe der Verschlusszapfen den Innenraum des Medikamentenreservoirs in mindestens zwei Teilvolumina unterteilt: ein erstes Teilvolumen, welches auf einer Seite von der Zerstäubermembran bzw. dem Aerosolerzeuger und auf einer anderen Seite durch eine Stirnfläche oder eine Teilfläche des Verschlusszapfen begrenzt wird, sowie mindestens ein weiteres, zweites Teilvolumen, welches den Rest des Reservoirs umfasst. Hierzu ist es nötig, dass der Verschlusszapfen bei verschlossener Kappe auf mindestens einer umlaufenden Linie an die Innenfläche des Reservoirs anliegt oder dieser zumindest sehr nahe kommt, sodass ein Außenquerschnitt des Verschlusszapfens mit einem Innenquerschnitt des Reservoirs (nahezu) deckungsgleich ist.

Dies kann beispielsweise dadurch erreicht sein, dass die Spitze des Verschlusszapfens als Teil eines Rotationskörpers, insbesondere als Kegel oder Kegelstumpf ausgeführt ist, und das Innere des Reservoirs eine in Form und Größe mindestens einem Teil dieses Rotationskörpers, insbesondere Kegelstumpfes, komplementäre Ausformung aufweist wobei sich diese beiden komplementären Teile im verschlossenen Zustand formschlüssig aneinander schmiegen.

Eine andere Möglichkeit, den Erfindungsgedanken umzusetzen, besteht darin, dass ein Teil der Innenfläche des Reservoirs eben ausgeformt und darin eine Öffnung vorhanden ist, deren Fläche ein auf einer anderen Seite von der Zerstäubermembran begrenztes Volumen begrenzt. Der Verschlusszapfen soll nun erfindungsgemäß so ausgestaltet sein, dass im verschlossenen Zustand eine Teilfläche des Verschlusszapfens, die in der ebenen Fläche vorhandene Öffnung vollständig bedeckt und verschließt. Um die Dichtigkeit des Abschlusses sicherzustellen, kann Verschlusszapfenseitig oder reservoirseitig eine Dichtung zum Beispiel in Form eines Dichtrings vorgesehen sein. Hierbei müssen weder Verschlusszapfen noch die Stufenfläche oder die Öffnung rotationssymmetrisch sein. Es kann zum Beispiel eine Fläche so vorgesehen sein, dass sie mit einer Drehachse der Kappe und des Verschlusszapfens nicht konzentrisch ist, je nach Verdrehung des Deckels die Öffnung freigegeben oder verschlossen ist.

Bei der Wahl der konkreten Form der komplementären Teile bzw. Teilflächen von Verschlusszapfen und Reservoirinnerem ist jedoch darauf zu achten, dass der beim Eintauchen des Verschlusszapfens in das Reservoir unweigerlich erzeugte Druck zumindest bei üblichen, axialen Eintauchgeschwindigkeiten zu allen Zeiten unter einem von der dünnen und darum recht empfindlichen Meshmembran des Aerosolerzeugers maximal tolerierbaren Druck liegt. Kritisch ist hierbei insbesondere die Zeit kurz vor der Herstellung des Formschlusses zwischen Verschlusszapfen(spitze) und Reservoirinnenfläche, wenn nur noch ein kleiner Spalt verbleibt, durch den überzählige Medikamentenflüssigkeit vom ersten ins zweite Teilvolumen verdrängt werden kann.

Um dieses Problem zu umgehen, schlägt eine Ausführungsform der vorliegenden Erfindung vor, dass die ineinandergreifenden komplementären Teilflächen des Verschlusszapfens und des Reservoirs sich auch bei fest verschlossener Kappe nicht berühren, sondern zwischen ihnen ein schmaler Spalt freibleibt. Dieser ist so bemessen, dass er einerseits groß, d.h. breit, genug ist, um ein problemloses Verdrängen der Flüssigkeit ohne allzu hohe Druckspitzen zu ermöglichen, andererseits aber klein, d.h. schmal, genug ist, um ein Nachströmen von Flüssigkeit vom zweiten in das sich im Laufe der Inhalation leerende erste Teilvolumen zu verhindern. Dies ist deshalb möglich, weil die Medikamentenflüssigkeit eine endliche Oberflächenspannung besitzt, so dass, solange der Spalt nicht zu weit ist, ein Flüssigkeitsrest darin verbleibt, der den Spalt wie eine Dichtung verschließt und das Nachströmen von Flüssigkeit effektiv verhindert. Hierbei ist auch wichtig, das die Abmessung der Kontaktfläche in axialer Richtung groß genug ist, in jedem Fall um Größenordnungen über der Weite des Spaltes, also dem Abstand der komplementären Teilflächen liegt. Variationen dieses Abstandes von Punkt zu Punkt sind dabei minimal zu halten, insbesondere ist es wichtig, dass der Abstand, also die Spaltweite an keinem Punkt zu groß wird. Was hierbei "zu groß" wäre, hängt hierbei im Wesentlichen von der konkreten Form der komplementären Flächen, der Oberflächenspannung der Flüssigkeit und dem während des Zerstäubens minimal erzeugten Unterdruck im ersten Teilvolumen ab.

Vorteilhafte Weiterbildungen vorliegender Erfindung, welche Einzeln oder in Kombination realisierbar sind, sofern sie sich nicht offensichtlich gegenseitig ausschließen, sollen im Folgenden beschrieben werden.

In einer bevorzugten Ausführungsform schlägt vorliegende Erfindung vor, dass der Verschlusszapfen im Wesentlichen als Zylinder ausgeführt, und, um Material und Gewicht zu sparen hohl gefertigt ist. Des Weiteren ist es zum Herstellen eines dichten Formschlusses mit einer entsprechend geformten Partie der Innenseite des Reservoirs vorteilhaft, wenn die Spitze des Verschlusszapfens eine kegel- oder kegelstumpfförmige Formgebung aufweist. Um die Unterteilung des Innenvolumens des Reservoirs in zwei Teilvolumina sicherzustellen, ist ein Querschnitt des Verschlusszapfens mit einem Innenquerschnitt des Reservoirs in Deckung gebracht. Dabei müssen diese beiden Querschnitte nicht rotationssymmetrisch sein, dies bietet sich jedoch an um in der Praxis einen zuverlässig, dichten Abschluss sicherzustellen.

Um die für einen zuverlässigen Abschluss nötigen Fertigungstoleranzen zu verringern, wird vorgeschlagen, die ineinandergreifenden, komplementären Teilflächen von Verschlusszapfen und/oder Reservoirinnenfläche mit einem elastischen Material zu versehen. Dies kann in Form eines in das Reservoir eingelegten oder um die Spitze des Verschlusszapfens gelegten Dichtrings realisiert sein. Gemäß der im Anspruch 1 definierten Erfindung sind eine oder beide der Teilflächen mit einem Überzug aus Gummi oder Silikon zu versehen.

Gemäß der im Anspruch 2 definierten Erfindung ist der gesamte Zapfen aus Silikon gefertigt. Diese Gestaltung hat den Vorteil, die nötigen einzuhaltenden Fertigungstoleranzen deutlich zu vergrößern, bei den üblichen Baugrößen von Verneblern etwa in den Bereich von einem halben Millimeter. Gerade bei der im Anspruch 2 definierten Erfindung umfasst der Verschlusszapfen als Versteifungsmerkmal in einer Längsrichtung des Verschlusszapfens verlaufende Versteifungsrippen, und/oder als Formhaltemerkmal quer zu der Längsrichtung verlaufende Formhalteringe.

Hierdurch wird effektiv verhindert, dass sich der Schaft des Zapfens durch ein seitliches Verbiegen derart verformt, dass der Flächenschluss der kooperierenden Teilflächen von Zapfenspitze und Medikamentenreservoir gefährdet ist.

Alternativ zu dem zuvor beschriebenen Abtrennen der beiden Teilvolumina mittels eines Formschlusses zwischen Verschlusszapfenaußen- und Reservoirinnenfläche schlägt eine Ausführungsform der vorliegenden Erfindung vor, dies mittels eines Flächenschlusses zwischen einer Stirnfläche beziehungsweise einer Seitenfläche des Verschlusszapfens und einer Stufenfläche, das heißt einer im Wesentlichen ebenen Fläche mit einer darin liegenden Öffnung, welche zum Aerosolerzeuger hinführt, zu bewerkstelligen. Um ein dichtes Verschließen zu gewährleisten, mag es nötig sein, entweder Verschlusszapfenseitig oder reservoirseitig eine Dichtung, zum Beispiel in Form eines flexiblen Kunststoffüberzugs oder eines Dichtrings, vorzusehen. Die Öffnung in der Stufenfläche kann hierbei symmetrisch zu einer Drehachse des Verschlusszapfens beziehungsweise der Kappe positioniert sein oder sie ist einer nicht symmetrischen Position gelegen, sodass bei Verdrehen eines ebenfalls nicht symmetrisch geformten Verschlusszapfens die Öffnung auch dann freigegeben wird, wenn keine axiale Verschiebung der Kappe erfolgt. Dies kann zum Beispiel realisiert werden, wenn der Verschluss der Kappe mittels eines Drehverschlusses erfolgt, bei dem am Ende des Drehbereiches das Gewinde eine verschwindende Steigung aufweist, sodass in diesem Drehbereich keine axiale Verschiebung mehr erfolgt.

Als weitere Verschlussmöglichkeiten, außer einem Drehverschluss bietet sich noch ein Schraub-, oder ein Bajonettverschluss an. Hierbei ist es zur Herstellung eines sicheren Verschlusses notwendig, dass Kappe und Reservoir eine gemeinsame Achse, die Drehachse besitzen.

Auf der Austrittsseite der Membran, bzw. des Aerosolerzeugers sollte sinnvollerweise entweder ein Mundstück ausgeformt sein oder eine Anschlussmöglichkeit für ein solches vorgesehen sein. Dieses Anschlussstück kann als Hohlzylinder mit geschlossenen Seitenflächen ausgeformt sein.

Es empfiehlt sich weiterhin, das Gesamtvolumen des Reservoirs abzüglich des minimalen Verschlusszapfenvolumens so groß zu wählen, dass es größer ist als das in üblichen Medikamententransportbehältern übliche Volumen.

Weitere Eigenschaften, Merkmale und Vorteile vorliegender Erfindung ergeben sich aus den im folgenden Anhang der Figuren näher erläuterten Ausführungsbeispiele, die aber nicht explizit alle Merkmale des Anspruchs 1 bzw. des Anspruchs 2 enthalten. Diese sollen die Erfindung nur illustrieren und in keiner Weise in ihrer Allgemeinheit einschränken.

Es zeigen:
- Figur 1:: Längsschnitt durch eine Basisversion der Verneblereinheit mit zweigeteiltem Medikamentenreservoir.
- Figur 2:: Längsschnitt durch eine Ausführungsform mit zweigeteiltem Medikamentenreservoir, bei dem die Größe des ersten Teilvolumens variierbar ist.
- Figur 3:: Zwei Längsschnitte durch eine Ausführungsform, bei dem ein Flächenschluss stattfindet.
- Figur 4:: In zwei Teilfiguren vorteilhafte Ausführungsformen des Verschlusszapfens des Verneblers

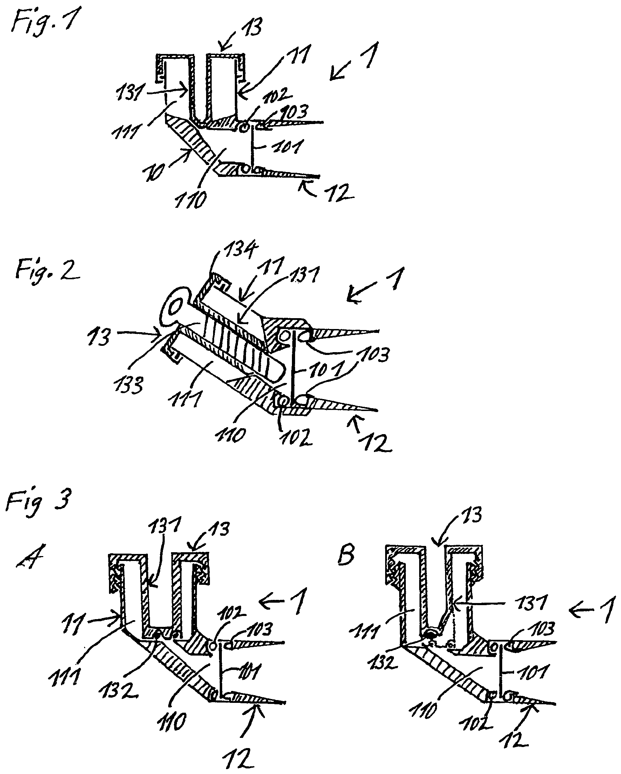

Figur 1 zeigt den Längsschnitt durch eine bevorzugte Ausführung der Verneblereinheit bei der ein zweigeteiltes Medikamentenreservoir vorliegt, wobei die Abtrennung der beiden Teilvolumina 110, 111 voneinander mittels eines Formschlusses zwischen der Spitze eines des zylinderförmigen Verschlusszapfens 131 und einer Komplementär ausgeformten Region des Reservoirs 11 stattfindet.

Zu sehen ist weiterhin das Gehäuse 10, welches den die Membran umfassenden Aerosolerzeuger 101, den Dichtring 102 und die Haltestruktur 103 umschließt und membranausgangsseitig einen Mundstückanschluss 12, sowie membraneingangsseitig ein Medikamentenreservoir 11 aufweist. Letzteres kann mittels einer fest schließenden Kappe 13 verschlossen werden, welche über den Verschlusszapfen 131 in Form eines Zylinders mit kegelstumpfförmiger Spitze verfügt. Im festverschlossenen Zustand der Kappe 13 ist wie abgebildet ein Formschluss zwischen dieser Kegelstumpfspitze des Verschlusszapfens 131 und einer Region im Innern des Reservoirs 11 hergestellt, sodass die Unterteilung in die beiden Teilvolumina 110, 111 erfolgt. Nach Beendigung einer Inhalation, das heißt wenn das erste Teilvolumen 110 vollständig geleert ist, kann eine zweite Inhalation mit ebenfalls exakter Medikamentendosierung erfolgen, indem einfach die Kappe 13 ganz oder teilweise abgenommen wird, sodass das Medikament aus dem zweiten Teilvolumen 111 in das erste Teilvolumen 110 nachströmen kann, und dann die Kappe 13 wieder fest aufgesetzt wird.

Figur 2 zeigt einen Längsschnitt durch eine andere Ausführungsform der Verneblereinheit, bei der ebenfalls ein Formschluss zwischen der Spitze des Verschlusszapfens 131 und einer Region des Medikamentenreservoirs 11 hergestellt wird. Zusätzlich zu der in Figur 1 gezeigten Ausführungsform ist jedoch die Kappe 13 zweiteilig ausgeführt. So besteht sie aus dem eigentlichen Deckel 134 und einem darin per Schraubgewinde einschraubbaren Bolzen 133, dessen Spitze mehr oder weniger weit in das erste Teilvolumen 110 hineinragen kann, sodass dessen verbleibendes, effektives Restvolumen variierbar ist. Wie bei der in Figur 1 gezeigten Verneblereinheit ist auch hier ein Anschluss 12 für ein Mundstück 2 vorgesehen.

Figur 3 zeigt in zwei Teilansichten Längsschnitte durch eine dritte bevorzugte Ausführungsform, bei der nicht ein Formschluss, sondern ein Flächenschluss zwischen einer Stirnfläche des Verschlusszapfens 131 sowie einer Stufenfläche im Innern des Reservoirs 11 erfolgt, welche mittels eines Verschlusszapfenseitig angebrachten Dichtungsrings 132 dicht abgeschlossen ist.

In dem in Teilfigur A gezeigten Schnitt ist die Kappe 13 mit Verschlusszapfen 131 fest auf das Medikamentenreservoir 11 aufgesetzt und verschlossen. Durch Flächenschluss zwischen einer Stirnfläche des Verschlusszapfens 131 mit einer Stufenfläche im Inneren des Reservoirs 11 wird eine Unterteilung in zwei, voneinander getrennte Teilvolumina 110 und 111 hergestellt. Die Dichtigkeit des Flächenschlusses ist durch einen Verschlusszapfenseitig befestigten Dichtungsring 132 sichergestellt.

Teilfigur B zeigt den Schnitt aus Teilfigur allerdings ist nun der Deckel 13 nicht fest verschlossen sondern um ca. 90Grad gegenüber dem in Teilfigur A gezeigten Zustand verdreht sowie aufgrund der Führung des Deckelrandes im Gewinde eines Schraubverschlusses leicht axial nach oben verschoben. Wie zu sehen ist, wird durch eine asymmetrische Formgebung des Verschlusszapfens 131, als Zylinder mit einer Abschrägung am unteren Ende, erreicht, dass in dieser Position die das Teilvolumen 110 begrenzende Öffnung in der Stufenfläche freigegeben wird. Die flüssigkeitsdichte Trennung der beiden Teilvolumina 110, 111 ist somit aufgehoben ein Flüssigkeitsaustausch kann gegebenenfalls stattfinden.

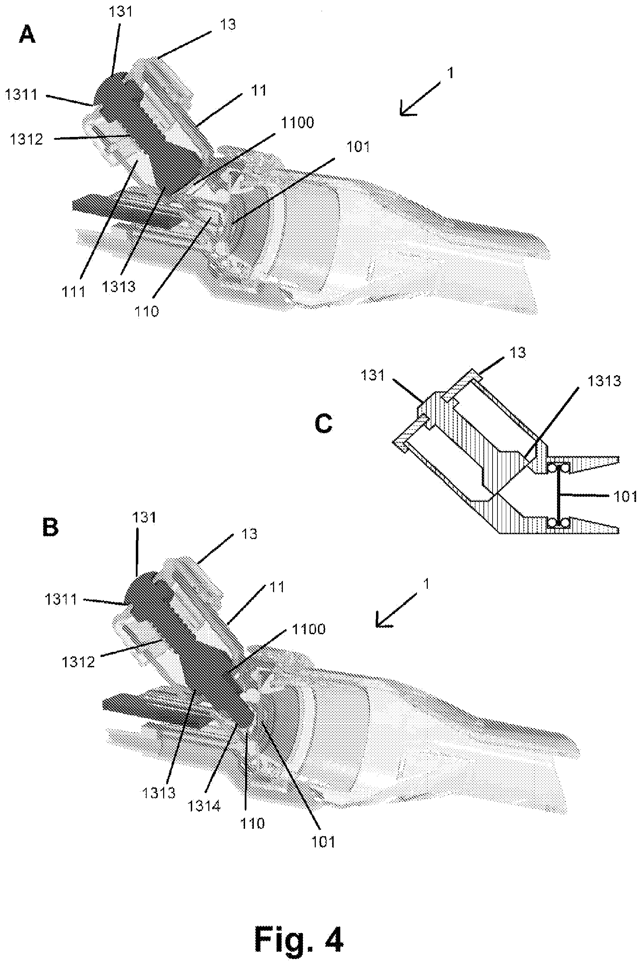

Figur 4 zeigt mögliche Ausgestaltungen eines einstückigen, massiven Verschlusszapfens.

Der Zapfen 131 aus Teilfigur A ist mittels eines doppelten Kragens 1311 in einer Öffnung in Kappe 13 gehalten. Der Schaft des Zapfens weist als Formgebungsmerkmale eine Reihe von quer zur Längsachse des Zapfens umlaufenden Wülsten 1312 auf. Diese bewirken, dass der Zapfen sich bei einer axialen Stauchung nicht so leicht verbiegt. Die den Verschluss von Öffnung 1100 bewirkende, mit der Innenfläche von Reservoir 11 kooperierende Teil 1313 des Zapfens 131 ist als sich nach oben verjüngender Kegelstumpf ausgebildet. Eine andere Formgebung, etwa mit einem gebogenen statt geraden Verlauf der Seiten wäre ebenfalls denkbar.

In Teilfigur B weist der Zapfen 131 darüber hinaus eine unten am Verschlussteil 1313 ansetzende Verdrängerspitze 1314 auf, die in das Teilvolumen 110 hineinragt und dessen Volumen entsprechend des Volumens der Spitze 1314 reduziert. Hierdurch können gegenüber der Ausführungsform aus Teilfigur A geringere Medikamentendosen verabreicht werden.

Teilfigur C zeigt einen schematischen Längsschnitt durch die Verneblereinheit aus Teilfigur A.

### Bezugszeichenliste

- 1: Verneblereinheit
- 10: Gehäuse
- 101: Aerosolerzeuger
- 102: Dichtring
- 103: Haltestruktur
- 11: Reservoir
- 110: Erstes Teilvolumen
- 1100: Öffnung
- 111: Weiteres Teilvolumen
- 12: Anschlussmöglichkeit für Mundstück
- 13: Kappe
- 131: Verschlusszapfen
- 1311: doppelter Kragen
- 1312: Formhalteringe
- 1313: Verschlussteil
- 1314: Verdrängerspitze
- 132: Dichtring
- 133: einschraubbarer Bolzen
- 134: Deckel
- 2: Mundstück

## Patentansprüche

1. Verneblereinheit mit Medikamentenreservoir zur Verwendung in einem Vernebler, umfassend
- einen Aerosolerzeuger (101) zur Zerstäubung einer Medikamentenflüssigkeit mit einer Zuführungsseite, die sich in Kontakt mit der dort vorhandenen Medikamentenflüssigkeit befindet und einer Ausgangsseite, auf der der gebildete Nebel abgegeben wird, und der zwischen einem zuführungsseitigen Dichtring (102) und einer ausgangseitigen Haltestruktur (103) gehalten ist,
- ein Gehäuse (10), welches Aerosolerzeuger (101), Dichtring (102) und Haltestruktur (103) einschließt und in welches auf der Zuführungsseite des Aerosolerzeugers (101) das
- Medikamentenreservoir (11) integriert ist, welches in seinem Inneren ein Hohlvolumen bereitstellt, in das die Medikamentenflüssigkeit eingebracht ist, und
- eine das Reservoir (11) dicht abschließende Kappe (13),
- wobei die Kappe (13) einen Verschlusszapfen (131) aufweist, der in den Innenraum des Reservoirs (11) reicht, und
- wobei ein Teil der Oberfläche des Verschlusszapfens (131) und ein Teil der Innenfläche des Reservoirs (11) komplementär zueinander geformt sind und bei fest aufgesetzter Kappe (13) einen flüssigkeitsdichten Verschluss bilden, durch den der Innenraum des Reservoirs (11) in ein erstes Teilvolumen (110), welches auf einer Seite von dem Aerosolerzeuger (101) und auf einer anderen Seite durch eine Stirn- oder eine Teilfläche des Verschlusszapfens begrenzt ist, sowie mindestens ein weiteres Teilvolumen (111) unterteilt ist, und
- wobei der flüssigkeitsdichte Verschluss dadurch zustande kommt, dass die komplementären Teilflächen nahe aneinander oder aufeinander zu liegen kommen oder gegeneinander gepresst werden, und
- wobei zumindest eine der beiden komplementären Teilflächen des Verschlusszapfens (131) und des Innenraums des Reservoirs (11) einen Überzug aus Gummi oder Silikon aufweist.

2. Verneblereinheit mit Medikamentenreservoir zur Verwendung in einem Vernebler, umfassend
- einen Aerosolerzeuger (101) zur Zerstäubung einer Medikamentenflüssigkeit mit einer Zuführungsseite, die sich in Kontakt mit der dort vorhandenen Medikamentenflüssigkeit befindet und einer Ausgangsseite, auf der der gebildete Nebel abgegeben wird, und der zwischen einem zuführungsseitigen Dichtring (102) und einer ausgangseitigen Haltestruktur (103) gehalten ist,
- ein Gehäuse (10), welches Aerosolerzeuger (101), Dichtring (102) und Haltestruktur (103) einschließt und in welches auf der Zuführungsseite des Aerosolerzeugers (101) das
- Medikamentenreservoir (11) integriert ist, welches in seinem Inneren ein Hohlvolumen bereitstellt, in das die Medikamentenflüssigkeit eingebracht ist, und
- eine das Reservoir (11) dicht abschließende Kappe (13),
- wobei die Kappe (13) einen Verschlusszapfen (131) aufweist, der in den Innenraum des Reservoirs (11) reicht, und
- wobei ein Teil der Oberfläche des Verschlusszapfens (131) und ein Teil der Innenfläche des Reservoirs (11) komplementär zueinander geformt sind und bei fest aufgesetzter Kappe (13) einen flüssigkeitsdichten Verschluss bilden, durch den der Innenraum des Reservoirs (11) in ein erstes Teilvolumen (110), welches auf einer Seite von dem Aerosolerzeuger (101) und auf einer anderen Seite durch eine Stirn- oder eine Teilfläche des Verschlusszapfens begrenzt ist, sowie mindestens ein weiteres Teilvolumen (111) unterteilt ist, und
- wobei der flüssigkeitsdichte Verschluss dadurch zustande kommt, dass die komplementären Teilflächen nahe aneinander oder aufeinander zu liegen kommen oder gegeneinander gepresst werden, und
- wobei der Verschlusszapfen (131)
• vollständig aus Silikon gefertigt ist, und
• als Versteifungsmerkmal in einer Längsrichtung des Verschlusszapfens (131) verlaufende Versteifungsrippen, und/oder als Formhaltemerkmal quer zu der Längsrichtung verlaufende Formhalteringe umfasst.

3. Verneblereinheit nach Anspruch 1 oder 2, **dadurch gekennzeichnet, dass** die komplementären Teilflächen des Verschlusszapfens (131) und des Reservoirs (11) sich auch bei fest verschlossener Kappe (13) nicht berühren, sondern zwischen ihnen ein schmaler Spalt freibleibt, der derart bemessen ist, dass er einerseits groß, d.h. breit, genug ist, um ein problemloses Verdrängen der Flüssigkeit ohne Druckspitzen zu ermöglichen, andererseits aber klein, d.h. schmal, genug ist, um ein Nachströmen von Medikamentenflüssigkeit vom weiteren Teilvolumen (111) in das sich im Laufe der Inhalation leerende erste Teilvolumen (110) zu verhindern.

4. Verneblereinheit nach einem der vorhergehenden Ansprüche, **dadurch gekennzeichnet, dass** der Verschlusszapfen (131)
- im Wesentlichen zylinderförmig ist, und/oder
- hohl oder massiv ist.

5. Verneblereinheit nach einem der vorhergehenden Ansprüche, **dadurch gekennzeichnet, dass** der Verschlusszapfen (131) ein rotationssymmetrisches, insbesondere kegel- oder kegelstumpfförmiges unteres Ende aufweist.

6. Verneblereinheit nach einem der vorhergehenden Ansprüche, **dadurch gekennzeichnet, dass** der Innenraum des Reservoirs (11) einen zumindest zu einem Teil der Spitze des Verschlusszapfens (131) komplementären Abschnitt enthält, dessen Innenfläche sich im aufgesetzten, vollständig verschlossenen Zustand der Kappe (13) umfänglich an die Spitze des Verschlusszapfens (131) formschlüssig anschmiegt, und so eine zumindest nahezu vollständige räumliche Trennung des ersten, auf einer Seite von dem Aerosolerzeuger (101) abgeschlossenen Teilvolumens (110) von einem zweiten Teilvolumen (111) erreicht ist.

7. Verneblereinheit nach einem der vorhergehenden Ansprüche, **dadurch gekennzeichnet, dass** im Innenraum des Reservoirs (11) eine im Wesentlichen ebene Stufenfläche vorhanden ist, welche eine Öffnung (1100) zu einem auf einer anderen Seite von dem Aerosolerzeuger (101) begrenzten Teilvolumen aufweist und der Verschlusszapfen (131) eine Stirnfläche aufweist, die größer ist als die Öffnung in der Stufenfläche und diese im verschlossenen Zustand vollständig bedeckt und verschließt.

8. Verneblereinheit nach dem vorgehenden Anspruch, **dadurch gekennzeichnet, dass** die Öffnung oder die Stirnfläche des Verschlusszapfens (131) relativ zu einer Drehachse der Kappe (13) so angeordnet sind, dass eine Drehung die Öffnung (1100) ganz oder teilweise freigibt.

9. Verneblerheinheit nach einem der vorhergehenden Ansprüche, **dadurch gekennzeichnet, dass** die Kappe (13) mittels eines Schraub-, Dreh- oder Bajonettverschluss auf dem Reservoir (11) lösbar befestigt ist.

10. Verneblereinheit nach einem der vorhergehenden Ansprüche, **dadurch gekennzeichnet, dass** das Reservoir (11) und die Kappe (13) eine gemeinsame Symmetrieachse besitzen.

11. Verneblereinheit nach einem der vorhergehenden Ansprüche, **dadurch gekennzeichnet, dass** der Verschlusszapfen (131) ein variables Volumen besitzt.

12. Verneblereinheit nach einem der vorhergehenden Ansprüche, **dadurch gekennzeichnet, dass** das Volumen des Verschlusszapfens (131) mittels eines in der Kappe in einem Gewinde geführten Bolzen variierbar ist.

## Claims

1. Nebulizer unit with medication reservoir for use in a nebulizer, comprising
- an aerosol generator (101) for atomizing a medication liquid having a feed side, which is in contact with the medication liquid present there, and a discharge side, at which the mist formed is emitted and which is retained between a sealing ring (102) at the supply side and a retaining structure (103) at the discharge side,
- a housing (10), which encloses the aerosol generator (101), the sealing ring (102) and the retaining structure (103) and into which, at the supply side of the aerosol generator (101), the
- medication reservoir (11) is integrated, which provides in its interior a hollow volume, into which the medication liquid is introduced, and
- a cap (13), which tightly seals the reservoir (11),
- the cap (13) having a closure pin (131), which extends into the interior space of the reservoir (11) and
- wherein a portion of the surface of the closure pin (131) and a portion of the inner surface of the reservoir (11) is formed complementary to one another and, when the cap (13) is firmly fitted, form a liquid-tight closure, by means of which the inner space of the reservoir (11) is subdivided into a first partial volume (110), which is delimited at one side by the aerosol generator (101) and on another side by an end surface or a partial surface of the closure pin, as well as at least one further partial volume (111), and
- wherein the liquid-tight closure is created by the fact that the complementary partial surfaces come to lie close to one another or one on top of the other or are pressed against one another, and
- wherein at least one of the two complementary partial surfaces of the closure pin (131) and of the inner space of the reservoir (11) has a rubber or silicone covering.

2. Nebulizer unit with medication reservoir for use in a nebulizer, comprising
- an aerosol generator (101) for atomizing a medication liquid having a feed side, which is in contact with the medication liquid present there, and a discharge side, at which the mist formed is emitted and which is retained between a sealing ring (102) at the supply side and a retaining structure (103) at the discharge side,
- a housing (10), which encloses the aerosol generator (101), sealing ring (102) and retaining structure (103) and into which, at the supply side of the aerosol generator (101), the
- medication reservoir (11) is integrated, which provides in its interior a hollow volume, into which the medication liquid is introduced, and
- a cap (13), which tightly seals the reservoir (11),
- the cap (13) having a closure pin (131), which extends into the interior space of the reservoir (11) and
- wherein a portion of the surface of the closure pin (131) and a portion of the inner surface of the reservoir (11) is formed complementary to one another and, when the cap (13) is firmly fitted, form a liquid-tight closure, by means of which the inner space of the reservoir (11) is subdivided into a first partial volume (110), which is delimited at one side by the aerosol generator (101) and on another side by an end face or a partial face of the closure pin, as well as at least one further partial volume (111), and
- wherein the liquid-tight closure is created by the fact that the complementary partial surfaces come to lie close to one another or one on top of the other or are pressed against one another, and
- wherein the closure pin (131)
• is made entirely of silicone and,
• as a stiffening feature, comprises stiffening ribs running in a longitudinal direction of the closure pin (131) and/or as shape-retaining feature, comprises shape-retaining rings running transverse to the longitudinal direction.

3. Nebulizer unit according to claim 1 or 2, **characterised in that,** the complementary partial surfaces of the closure pin (131) and of the reservoir (11) are not in contact with one another even when the cap is firmly closed (13), but a narrow gap remains open between them, which is dimensioned such that on the one hand it is large, i.e. wide, enough, to allow smooth displacement of the liquid without pressure spikes, but on the other hand it is small, i.e. narrow, enough to prevent inflow of medicament liquid from the further partial volume (111) into the first partial volume (110) which is being emptied in the course of the inhalation.

4. Nebulizer unit according to one of the preceding claims, **characterised in that** the closure pin (131)
- is essentially cylindrical, and/or
- is hollow or solid.

5. Nebulizer unit according to one of the preceding claims, **characterised in that** the closure pin (131) comprises a rotationally symmetrical, in particular a conical or truncated-conical lower end.

6. Nebulizer unit according to one of the preceding claims, **characterized in that** the inner space of the reservoir (11) contains a section that is complementary to at least a portion of the tip of the closure pin (131), of which section the inner surface of the cap (13), when completely closed, conforms in a form-fitting manner circumferentially to the tip of the closure pin, and thus achieves an at least almost complete spatial separation of the first partial volume (110), which is closed off at one side by the aerosol generator (101), from a second partial volume (111).

7. Nebulizer unit according to one of the preceding claims, **characterized in that** in the interior space of the reservoir (11), an essentially planar stepped surface is present, which has an opening (1100) to a partial volume that is delimited at a different side by the aerosol generator (101), and the closure pin (131) has an end surface that is larger than the opening in the stepped surface and covers and seals the latter when closed.

8. Nebulizer unit according to the preceding claim, **characterised in that,** the opening or the end surface of the closure pin (131) is disposed relative to an axis of rotation of the cap (13), such that a rotation entirely or partly releases the opening (1100).

9. Nebulizer unit according to one of the preceding claims, **characterised in that,** the cap (13) is detachably fixed on the reservoir (11) by means of a screw, turn or bayonet lock.

10. Nebulizer unit according to one of the preceding claims, **characterised in that** the reservoir (11) and the cap (13) possess a common axis of symmetry.

11. Nebulizer unit according to one of the preceding claims, **characterised in that** the closure pin (131) has a variable volume.

12. Nebulizer unit according to one of the preceding claims, **characterised in that** the volume of the closure pin (131) is variable by means of a bolt that is guided in a thread in the cap.

## Revendications

1. Unité de nébulisation avec un réservoir de médicament destiné à être utilisé dans un nébuliseur, comprenant
- un générateur d'aérosol (101) pour atomiser un liquide médicamenteux ayant un côté alimentation, qui est en contact avec le liquide médicamenteux qui s'y trouve, et un côté décharge, au niveau duquel le brouillard formé est émis et qui est retenu entre une bague d'étanchéité (102) du côté de l'alimentation et une structure de retenue (103) du côté de la décharge,
- un boîtier (10) qui renferme le générateur d'aérosol (101), la bague d'étanchéité (102) et la structure de retenue (103) et dans lequel, du côté alimentation du générateur d'aérosol (101)
- un réservoir de médicament (11) est intégré, qui fournit un volume creux à l'intérieur dans lequel le liquide médicamenteux est introduit, et
- un capuchon (13) fermant hermétiquement le réservoir (11),
- dans lequel le capuchon (13) a un tourillon de fermeture (131) qui s'étend à l'intérieur du réservoir (11), et
- dans lequel une partie de la surface du tourillon de fermeture (131) et une partie de la surface intérieure du réservoir (11) sont conformées de façon complémentaire l'une à l'autre et forment un joint étanche aux liquides lorsque le capuchon (13) est fermement en place, par lequel l'intérieur du réservoir (11) est subdivisé en un premier volume partiel (110), qui est délimité d'un côté par le générateur d'aérosol (101) et de l'autre côté par une face d'extrémité ou une surface partielle du tourillon de fermeture, et en au moins un autre volume partiel (111), et
- dans lequel le joint étanche aux liquides est réalisé par le fait que les surfaces partielles complémentaires viennent se trouver à proximité l'une de l'autre ou se superposer ou sont pressées l'une contre l'autre, et
- dans lequel au moins l'une des deux surfaces partielles complémentaires du tourillon de fermeture (131) et l'intérieur du réservoir (11) présente un revêtement en caoutchouc ou en silicone.

2. Unité de nébulisation avec un réservoir de médicament destiné à être utilisé dans un nébuliseur, comprenant
- un générateur d'aérosol (101) pour atomiser un liquide médicamenteux ayant un côté alimentation, qui est en contact avec le liquide médicamenteux qui s'y trouve, et un côté décharge, au niveau duquel le brouillard formé est émis et qui est retenu entre une bague d'étanchéité (102) du côté de l'alimentation et une structure de retenue (103) du côté de la décharge,
- un boîtier (10) qui renferme le générateur d'aérosol (101), la bague d'étanchéité (102) et la structure de retenue (103) et dans lequel, du côté alimentation du générateur d'aérosol (101)
- un réservoir de médicament (11) est intégré, qui fournit un volume creux à l'intérieur dans lequel le liquide médicamenteux est introduit, et
- un capuchon (13) fermant hermétiquement le réservoir (11),
- dans lequel le capuchon (13) a un tourillon de fermeture (131) qui s'étend à l'intérieur du réservoir (11), et
- dans lequel une partie de la surface du tourillon de fermeture (131) et une partie de la surface intérieure du réservoir (11) sont conformées de façon complémentaire l'une à l'autre et forment un joint étanche aux liquides lorsque le capuchon (13) est fermement en place, par lequel l'intérieur du réservoir (11) est subdivisé en un premier volume partiel (110), qui est délimité d'un côté par le générateur d'aérosol (101) et de l'autre côté par une face d'extrémité ou une surface partielle du tourillon de fermeture, et en au moins un autre volume partiel (111), et
- dans lequel le joint étanche aux liquides est réalisé par le fait que les surfaces partielles complémentaires viennent se trouver à proximité l'une de l'autre ou se superposer ou sont pressées l'une contre l'autre, et
- dans lequel le tourillon de fermeture (131)
• est entièrement en silicone, et
• comprend, en tant qu'élément de renforcement, des nervures de renforcement s'étendant dans une direction longitudinale du tourillon de fermeture (131), et/ou, en tant qu'élément de maintien de forme, des anneaux de maintien de forme s'étendant transversalement à la direction longitudinale.

3. Unité de nébulisation selon la revendication 1 ou 2, **caractérisé en ce que** les surfaces partielles complémentaires du tourillon de fermeture (131) et du réservoir (11) ne sont pas en contact l'une avec l'autre même lorsque le capuchon est hermétiquement fermé (13), mais un espace étroit reste ouvert entre eux, qui est dimensionné de telle sorte que d'une part il soit grand, c'est-à-dire large, suffisamment pour permettre au liquide de se déplacer sans problème et sans pics de pression, mais d'un autre part il est petit, c'est-à-dire étroit, suffisamment pour empêcher le reflux du liquide médicamenteux de l'autre volume partiel (111) dans le premier volume partiel (110) qui est vidé au cours de l'inhalation.

4. Unité de nébulisation selon l'une des revendications précédentes, **caractérisée en ce que** le tourillon de fermeture (131)
- est essentiellement cylindrique, et/ou
- est creux ou plein.

5. Unité de nébulisation selon l'une des revendications précédentes, **caractérisé en ce que** le tourillon de fermeture (131) comporte une extrémité inférieure à symétrie de révolution, notamment conique ou tronconique.

6. Unité de nébulisation selon l'une des revendications précédentes, **caractérisé en ce que** l'intérieur du réservoir (11) contient une section complémentaire d'au moins une partie de l'embout du tourillon de fermeture (131), dont la section la surface interne du capuchon (13), lorsqu'il est complètement fermé, épouse la forme circonférentiellement de la pointe de l'ergot de fermeture, et réalise ainsi une séparation spatiale au moins presque complète du premier volume partiel (110), qui est fermé d'un côté par le générateur d'aérosol (101), à partir d'un second volume partiel (111).

7. Unité de nébulisation selon l'une des revendications précédentes, **caractérisée en ce que** dans l'intérieur du réservoir (11), une surface étagée essentiellement plane est présente, qui présente une ouverture (1100) vers un volume partiel qui est délimité à un côté différent par le générateur d'aérosol (101), et le tourillon de fermeture (131) a une surface d'extrémité qui est plus grande que l'ouverture dans la surface étagée et recouvre et scelle cette dernière lorsqu'elle est fermée.

8. Unité de nébulisation selon la revendication précédente, **caractérisé en ce que** l'ouverture ou la surface d'extrémité du tourillon de fermeture (131) est disposée par rapport à un axe de rotation du capuchon (13), de sorte qu'une rotation libère totalement ou partiellement le ouverture (1100).

9. Unité de nébulisation selon l'une des revendications précédentes, **caractérisée en ce que**, le capuchon (13) est fixé de manière amovible sur le réservoir (11) au moyen d'un verrouillage à vis, à tour ou à baïonnette.

10. Unité de nébulisation selon l'une des revendications précédentes, **caractérisé en ce que** le réservoir (11) et le capuchon (13) possèdent un axe de symétrie commun.

11. Unité de nébulisation selon l'une des revendications précédentes, **caractérisée en ce que** la tourillon de fermeture (131) a un volume variable.

12. Unité de nébulisation selon l'une des revendications précédentes, **caractérisée en ce que** le volume du tourillon de fermeture (131) est variable au moyen d'un boulon qui est guidé dans un filetage dans le capuchon.
